(19) Europäisches Patentamt
European Patent Office
Office européen des brevets

(11) Veröffentlichungsnummer: **0 241 664 B1**

(12) # EUROPÄISCHE PATENTSCHRIFT

(45) Veröffentlichungstag der Patentschrift: **02.08.89**

(51) Int. Cl.[4]: **C07C 37/60**, C07C 65/03, C07C 65/05, C07C 51/367, C07C 69/88, C07C 43/178, C07C 79/26, C07C 87/50, C07C 39/24, C07C 37/00, C07C 51/00

(21) Anmeldenummer: **87101957.6**

(22) Anmeldetag: **12.02.87**

(54) **Verfahren zur Herstellung substituierter Trihydroxybenzole.**

(30) Priorität: **11.03.86 DE 3607923**

(43) Veröffentlichungstag der Anmeldung: **21.10.87 Patentblatt 87/43**

(45) Bekanntmachung des Hinweises auf die Patenterteilung: **02.08.89 Patentblatt 89/31**

(84) Benannte Vertragsstaaten: **AT CH DE FR GB GR IT LI NL SE**

(56) Entgegenhaltungen:
**DE-A- 1 228 258**
**DE-A- 2 064 497**
**DE-A- 2 410 758**

(73) Patentinhaber: **Degussa Aktiengesellschaft, Weissfrauenstrasse 9, D-6000 Frankfurt am Main 1(DE)**

(72) Erfinder: **Prescher, Günter, Dr., Liesingstrasse 2, D-6450 Hanau 9(DE)**
Erfinder: **Ritter, Gebhard, Dr., Fürstenbergstrasse 2, D-6450 Hanau 9(DE)**
Erfinder: **Sauerstein, Holger, Goethestrasse 1, D-6451 Grosskrotzenburg(DE)**

EP 0 241 664 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von Mischungen substituierter 1,2,3- und 1,2,4-Trihydroxybenzole.

Die genannten Verbindungen sind wichtige Synthesebausteine für Produkte in der Pharmazie, für Kosmetik, Agro- und Fotochemikalien (Kirk Othmer, Encyclopedia Chem. Technol., 3. Auflage, Band 18, Seite 670 - 84, 1982).

Bisher konnten diese Verbindungen nur sehr aufwendig über mehrere Verfahrensstufen hergestellt werden.

Nach der EU-PS 25 659 wurden substituierte Pyrogallolverbindungen zunächst z.B. durch Chlorierung von 2,6-Dimethylphenolabkömmlingen in entsprechende Chlorverbindungen überführt, deren Chlormethylgruppen dann in Aldehydgruppen umgewandelt wurden. Die Aldehyde wurden entsprechend der Dakin-Reaktion mit Perverbindungen, wie Wasserstoffperoxid oder Persäuren bzw. deren Salzen, in basischem Medium oxydiert zu den gewünschten Pyrogallolverbindungen. Dieses mehrstufige Verfahren führte trotz des Aufwandes nur zu mäßigen Ausbeuten.

Ein weiteres mehrstufiges Verfahren über eine modifizierte Dakin-Reaktion, die über Hydroxybenzaldehyde verläuft, führte nur dann zu zufriedenstellenden Ausbeuten, wenn der pH-Wert während der Reaktion kontinuierlich auf 6 durch Basenzugabe gehalten wurde. Hierdurch wurde auch die Aufarbeitung erschwert (EU-PS 44 260).

Ein ebenfalls umständliches Verfahren mit sehr schwankenden Ausbeuten an Pyrogallol oder Pyrogallolverbindungen wird in der DE-OS 2 653 446 beschrieben. Ein 2,2,6,6-Tetrahalogencyclohexanon muß zunächst hergestellt werden, das dann mit Wasser in Gegenwart eines Katalysators oder in Form eines Alkoxyds mit Säure hydroliysiert wird.

Eine weitere Möglichkeit, über die Thiele-Winter-Reaktion zu Hydroxyhydrochinonen bzw. substituierten Polyhydroxyverbindungen zu kommen, war gleichzeitig sehr umständlich und technisch aufwendig. Die Reaktion besteht ja in der Umwandlung eines entsprechenden Chinons in ein Hydroxyhydrochinon durch dessen Acetoxylierung mit Essigsäureanhydrid in Gegenwart von Schwefelsäure oder Borfluoridätherat zu dem entsprechenden Triacetat und dann dessen Umwandlung in das gewünschte Hydroxyhydrochinon bzw. in substituierte Polyhydroxyverbindungen (DE-OS 2 459 059).

Die DE-OS 2 064 497 lehrt die Hydroxylierung von Phenol, $C_1$- bis $C_4$-Alkyl substituiertem Phenol sowie deren $C_1$- bis $C_4$-Alkylethern mit Wasserstoffperoxid in einem Medium, das zu Beginn der Umsetzung weniger als 20 Gew.-% Wasser enthält, wobei für die Reaktion die Anwesenheit einer katalytischen Menge einer starken Säure zwingend ist. Bei abnehmendem $H^+/H_2O_2$-Verhältnis sinkt die Ausbeute trotz verlängerter Reaktionszeit.

Aus der DE-AS 1 228 258 ist ein Verfahren zur Ringhydroxylierung aromatisoher Verbindungen mit wenigstens einem elektronenanziehenden Substituenten und wenigsten einer Hydroxygruppe bekannt, wobei die Umsetzung mit Wasser und/oder Wasserstoffperoxid unter Bestrahlung mit energiereichen Strahlen, wie Co-60-Strahlen, erfolgt. Dieses Verfahren erfordert einen hohen technischen Aufwand für die Strahlenquelle und Schutzeinrichtungen hierfür.

Aufgabe der Erfindung ist es daher, substituierte 1,2,3- und 1,2,4-Trihydroxybenzole aus den entsprechend substituierten Resorcinen durch Hydroxylierung mit Wasserstcffperoxid mit geringerem technischen Aufwand herzustellen, als dies bisher möglich war.

Es wurde gefunden, daß sich diese Aufgabe lösen läßt, wenn man substituierte Resorcine der Formel

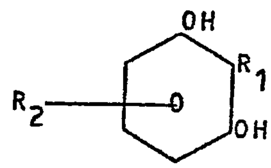

in der $R_1$ und $R_2$ Wasserstoff, aliphatische gesättigte, geradkettige oder verzweigte Kohlenwasserstoffreste mit 1–6 C-Atomen, die Gruppe –COOH, –$COOR_3$, –$CH_2OR_3$, –$SO_3H$, –$NO_2$, $NH_2$ oder F, Cl, Br, J bedeuten, $R_3$ einen aliphatischen gesättigten, geradkettigen oder verzweigten Kohlenwasserstoffrest mit 1–6 C-Atomen darstellt und $R_1$ und $R_2$ gleich oder verschieden sein können, jedoch beide nicht gleichzeitig Wasserstoff sind, ohne Zusatz von Katalysatoren und ohne Anwendung energiereicher Strahlung mit Wasserstoffperoxid im Molverhältnis 2 bis 20 : 1 bei Temperaturen von 60 bis 150°C in Kontakt bringt, wobei man wäßriges Wasserstoffperoxid mit einer Konzentration von 20–85 Gew.-% einsetzt und die Wassermenge des vor Beginn der Reaktion vorliegenden Gemisches zwischen 0,1 und 36 Gew.-%, bezogen auf das Gemisch, liegt.

Resorcine, die für die Erfindung zum Beispiel in Frage kommen, sind 2-Methylresorcin; 5-Methylresorcin (Orcin); 4n-Hexylrlesorcin; 2,5-Dimenthylresorcin (β-Orcin); 4-Chlorresorcin; 4,6-Dichlorresorcin, Dihydroxybenzosäure. Bevorzugt sind 2-Methylresorcin, Dihydroxybenzosäure. Resorcin selbst wird im erfindungsgemäßen Verfahren nicht verwendet.

Die einzusetzenden Resorcine liegen im allgemeinen in käuflicher Reinheit vor. Sie werden bevorzugt als Schmelze eingesetzt, solange ihre Schmelzpunkte so niedrig sind, daß sie mit Wasserstoffperoxid keine sicherheitsbedenklichen Mischungen bilden.

Haben die Resorcine Schmelzpunkte, die in der Nähe und oberhalb des Siedepunktes von Wasserstoffperoxid liegen, so sollten sie in Form ihrer Lösungen in Wasser oder in organischen Lösungsmitteln verwendet werden.

Die Auswahl der organischsen Lösungsmittel sollte so erfolgen, daß ihre Siedepunkte in der Nähe der gewünschten Arbeitstemperatur liegen.

Gleichgültig, ob wäßrige Lösungen der Resorcine oder Lösungen in organischen Lösungsmitteln vorliegen, bevorzugt werden ihre gesättigten Lösungen verwendet, um die Reaktionsgeschwindigkeit nicht unnötig herabzusetzen.

Als Lösungsmittel kommen übliche Lösungsmittel, wie aliphatische Carbonsäureester, z.B. Essigsäureäthyl-, propyl-, n-butyl-, sek-butyl-, tert.butyl-, n-hexylester in Frage, außerdem chlorierte Kohlenwasserstoffe wir z.B. Chloroform, Tetrachlorkohlenstoff, Methylenchlorid oder aromatische Kohlenwasserstoffe, wie Benzol oder Toluol.

Die wäßrigen Wasserstoffperoxide sind handelsübliche Lösungen mit Konzentrationen von 20–85 Gewichtsprozent Wasserstoffperoxid, bevorzugt von 50–85 Gewichtsprozent.

Günstige Anfangstemperaturen bei Inkontaktbringen von dem betreffenden Resorcin und Wasserstoffpeoxid sind 90–120°C.

Bevorzugte Wassermengen vor Beginn der Reaktion liegen bei 0,5 bis 15 Gewichtsprozent, besonders bevorzugt bei 0,5 bis 10 Gewichtsprozent, bezogen auf das vor Beginn der Reaktion vorliegen Gemisch. Ganz besonders bevorzugt sind Wassermengen von 0,5 bis 6 Gew.-%.

Das Molverhältnis von Resorcin zu Wasserstoffperoxid liegt bei 2 bis 20 : 1, bevorzugt bei 5 bis 10 : 1.

Der technische Fortschritt des erfindungsgemäßen Verfahrens liegt in der Möglichkeit, ausgehend von handelsüblichen Ausgangsstoffen, mit geringem technischen Aufwand zu den gewünschten 1,2,3- und 1,2,4-Trihydroxybenzolen zu kommen.

Es kommt hinzu, daß die Reaktionsdauer bei der eigentlichen Umsetzung wesentlich verkürzt ist und trotzdem günstige Ausbeuten erhalten werden.

Wurster erhielt im Gegensatz zum erfindungsgemäßen Verfahren durch Umsetzung von Orcin mit wäßrigem Wasserstoffperoxid in Gegenwart von kohlensaurem Natron eine Farbstoffbildung, nämlich ein Scharlach bzw. Bordeaux und in Gegenwart von Ammoniak rotviolette Farbstoffe (Berichte 10 (1887) Seite 2939).

Die Erfindung wird an den folgenden Beispielen näher erläutert.

Beispiel 1

38,53 g (0,25 mol) 2,6-Dihydroxybenzoesäure werden in 30 g n-Propylacetat aufgelöst und bis zum Sieden auf 110 °C erwärmt. Zu dieser gerührten Lösung gibt man 2,76 g (0,055 mol) 70 %iges Wasserstoffperoxid.

Die Temperatur in der Reaktionslösung erhöht sich danach auf 120 °C. Nach Abklingen der Exotherme wird nach 10 Minuten ein Wasserstoffperoxid-Umsatz von 100 % bestimmt. Das Reaktionsgemisch enthält dann 6,9 g (0,040 mol) 2,3,6-Trihydroxybenzoesäure, was einer Gesamtausbeute von 74,7 %, bezogen auf eingesetztes Wasserstoffperoxid, entspricht.

Beispiel 2

12,4 g (0,1 mol) 2-Methylresorcin werden auf 120 °C erwärmt. Zu dieser gerührten Schmelze gibt man 1,25 g (0,025 mol) 70 %iges Wasserstoffperoxid.

Die Temperatur in der Reaktionslösung erhöht sich danach auf 150 °C. Nach Abklingen der Exotherme wird nach 10 Minuten ein Wasserstoffperoxid-Umsatz von 100 % bestimmt. Das Reaktionsgemisch enthält dann 2,56 g (0,018 mol) 2,3,6-Trihydroxytoluol, was einer Gesamtausbeute von 73,2 %, bezogen auf eingesetztes Wasserstoffperoxid, entspricht.

**Patentansprüche**

1. Verfahren zur Herstellung von substituierten 1,2,3- und 1,2,4-Trihydroxydbenzolen durch Hydroxylierung von substituierten Resorcinen mit Wasserstoffperoxid, dadurch gekennzeichnet, daß man substituierte Resorcine der Formel

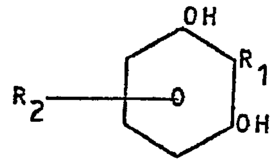

in der $R_1$ und $R_2$ Wasserstoff, aliphatische gesättigte, geradkettige oder verzweigte Kohlenwasserstoffreste mit 1–6 C-Atomen, die Gruppe $-COOH$, $-COOR_3$, $-CH_2OR_3$, $-SO_3H$, $-NO_2$, $NH_2$ oder F, Cl, Br, J bedeuten, $R_3$ einen aliphatischen gesättigten, geradkettigen odere verzweigten Kohenwasserstoffrest mit 1–6 C-Atomen darstellt und $R_1$ und $R_2$ gleich oder verschieden sein können, jedoch beide nicht gleichzeitig Wasserstoff sind, ohne Zusatz von Katalysatoren und ohne Anwendung energiereicher Strahlung mit Wasserstoffperoxid im Molverhältnis 2 bis 20 : 1 bei Temperaturen von 60 bis 150°C in Kontakt bringt, wobei man wäßriges Wasserstoffperoxid mit einer Konzentration von 20 – 85 Gew.-% einsetzt und die Wassermenge des vor Beginn der Reaktion vorliegenden Gemisches zwischen 0,1 und 36 Gew.-% , bezogen auf das Gemisch liegt.

2. Verfahren nach Anspruch 1, dadurch gekennzeichnet, daß man 2-Methylresorcin; 5-Methylresorcin; 4n-Hexylresorcin; 2,5-Dimethylresorcin; 4-Chlorresorcin; 4,6-Dichlorresorcin oder Dihydroxybenzoesäure einsetzt.

3. Verfahren nach Anspruch 2, dadurch gekennzeichnet, daß man 2-Methylresorcin in Form seiner Schmelze einsetzt.

4. Verfahren nach einem der Ansprüche 1 bis 3, dadurch gekennzeichnet, daß man das substituierte Resorcin und Wasserstoffperoxid bei 90–120°C miteinander in Kontakt bringt.

5. Verfahren nach einem oder mehreren den Ansprüche 1 bis 4, dadurch gekennzeichnet, daß die Wassermenge des vor Beginn der Reaktion vorliegenden Gemisches 0,5–15 Gew.-%, bevorzugt 0,5 bis 6 Gew.-%, beträgt.

6. Verfahren nach einem oder mehreren der Ansprüche 1 bis 5, dadurch gekennzeichnet, daß man das substituierte Resorcin und Wasserstoffperoxid im Molverhältnis von 2 bis 10 : 1 einsetzt.

7. Verfahren nach einem oder mehreren der Ansprüche 1 bis 6, dadurch gekennzeichnet, daß man

wäßriges Wasserstoffperoxid mit einer Konzentration von 50–85 Gew.-% einsetzt.

## Claims

1. A process for the production of substituted 1,2,3- and 1,2,4-trihydroxybenzenes by hydroxylation of substituted resorcinols with hydrogen peroxide, characterized in that substituted resorcinols corresponding to the following formula

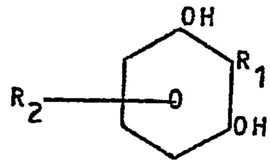

in which $R_1$ and $R_2$ represent hydrogen, aliphatic, saturated, linear or branched $C_1$–$C_6$-hydrocarbon radicals, the group $-COOH$, $-COOR_3$, $-CH_2OR_3$, $-SO_3H$, $-NO_2$, $NH_2$ or F, Cl, Br, I, $R_3$ is an aliphatic, saturated, linear or branched $C_1$–$C_6$ hydrocarbon radical and $R_1$ and $R_2$ may be the same or different, but are not both hydrogen, are contacted with hydrogen peroxide in a molar ratio of 2 to 20 : 1 at temperatures in the range from 60 to 150°C in the absence of catalysts and in the absence of high-energy radiation, aqueous hydrogen peroxide with a concentration of 20 to 85% by weight and the quantity of water in the mixture before the beginning of the reaction being from 0.1 to 36% by weight, based on the mixture.

2. A process as claimed in claim 2, characterized in that 2-mehtyl resorcinol; 5-methyl resorcinol; 4n-hexyl resorcinol; 2,5-dimethyl resorcinol; 4-chlororesorcinol; 4,6-dichlororesorcinol or dihydroxybenzoic acid are used.

3. A process as claimed in claim 2, characterized in that 2-methyl resorcinol is used in the form of a melt.

4. A process as claimed in any of claims 1 to 3, characterized in that the substituted resorcinol and hydrogen peroxide are contacted with one another at 90 to 120°C.

5. A process as claimed in one or more of claims 1 to 4, characterized in that the quantity of water in the mixture before the beginning of the reaction is from 0.5 to 15% by weight and preferably from 0.5 to 6% by weigth.

6. A process as claimed in one or more of claims 1 to 5, characterized in that the substituted resorcinol and hydrogen peroxide are used in a molar ratio of 2 to 10 : 1.

7. A process as claimed in one or more of claims 1 to 6, characterized in that aqueous hydrogen peroxide with a concentration of 50 to 85% by weight is used.

## Revendications

1. Procédé de préparation de 1,2,3- et 1,2,4-trihydroxybenzènes substitués par hydroxylation de résorcinols substitués avec du peroxyde d'hydrogène, caractérisé en ce qu'on met en contact avec du peroxyde d'hydrogène en proportion molaire de 2 à 20 : 1 à des températures comprises entre 60 et 150ÉC des résorcinols substitués de formule:

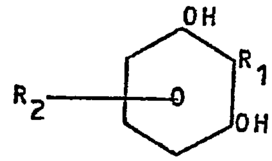

dans laquelle $R_1$ et $R_2$ sont l'hydrogène, des restes hydrocarbures aliphatiques saturés, linéaires ou ramifiés avec 1–6 atomes de C, les groupes $-COOH$, $-COOR_3$, $-CH_2OR_3$, $-SO_3H$, $-NO_2$, $NH_2$ ou F, Cl, Br, I, $R_3$ est un reste hydrocarboné aliphatique saturé, linéaire ou ramifié avec 1–6 atomes de C, et $R_1$ et $R_2$ peuvent être identiques ou différentes, cependant ils ne peuvent être simultanément l'hydrogène, sans addition de catalyseurs, ni utilisation de rayonnement énergétique, en utilisant de la solution aqueuse de peroxyde d'hydrogène à une concentration de 20–85% en poids, et la quantité d'eau du mélange présent avant le début de la réaction se situe entre 0,1 et 36% en poids, rapporté au mélange.

2. Procédé selon la revendication 1, caractérisé en ce qu'on utilise de 2-méthylrésorcinol; 5-méthylrésorcinol; 4n-hexylrésorcinol; 2,5-diméthylrésorcinol; 4-chlororésorcinol; 4,6-dichlororésorcinol ou de l'acide dihydroxybenzoïque.

3. Procédé selon la revendication 2, caractérisé en ce qu'on utilise le 2-méthylrésorcinol sous forme de sa masse fondue.

4. Procédé selon l'une des revendications 1 à 3, caracérisé en ce qu'on met en contacct le résorcinol substitué et le peroxyde d'hydrogène à 90–120°C.

5. Procédé selon l'une ou plusieurs des revendications 1 à 4, caractérisé en ce que la quantité d'eau du mélange présent avant le début de la réaction vaut 0,5–15% en poids, de préférence 0,5 à 6% en poids.

6. Procédé selon l'une ou plusieurs des revendications 1 à 5, caractérisé en ce qu'on utilise le résorcinol substitué et le peroxyde d'hydrogène dans une proportion molaire de 2 à 10 : 1.

7. Procédé selon l'une ou plusieurs des revendications 1 à 6, caractérisé en ce qu'on utilise une solution aqueuse de peroxyde d'hydrogène avec une concentration de 50–85% en poids.